Europäisches Patentamt

European Patent Office

Office européen des brevets

(11) Publication number: **0 276 575 B1**

## EUROPEAN PATENT SPECIFICATION

(45) Date of publication of patent specification: **04.03.92**

(51) Int. Cl.5: **A01G 7/00**

(21) Application number: **87311472.2**

(22) Date of filing: **24.12.87**

Divisional application 91113528.3 filed on 24/12/87.

(54) **Method of multiplicating plant seedlings.**

(30) Priority: **26.12.86 JP 308539/86**
**26.12.86 JP 308540/86**
**26.06.87 JP 157809/87**

(43) Date of publication of application:
**03.08.88 Bulletin 88/31**

(45) Publication of the grant of the patent:
**04.03.92 Bulletin 92/10**

(84) Designated Contracting States:
**BE DE FR GB IT NL**

(56) References cited:

**DERWENT JAPANESE PATENTS GAZETTE, Section Ch, Week 8742, no. 87-295978, 1987, Derwent Publications Ltd, London, GB; & JP-A-62 208 220 MITSUI PETROCHEM IND. K.K.) 12-09-1987**

**CHEMICAL ABSTRACTS, vol. 67, no. 17, 23rd October 1967, page 7504, abstract no. 79730c, Columbus, Ohio, US; NELLO BAGNI: "Absorption of spermine and their degradation in explants of Helianthus tuberosus in vitro", & Z. PFLANZENPHYSIOL. 57(1), 22-5 (1967)**

**CHEMICAL ABSTRACTS, vol. 97, no. 15, 11th October 1982, page 400, abstract no. 124038r, Columbus, Ohio, US; V.I. OSH-MARINA et al.: "Changes in the content of free amino acids and amides in Nicotiana sylvestris cell culture in response to various concentrations of putrescine in the medium", & FIZIOL. RAST. (MOSCOW) 1982, 29(4), 633-8**

(73) Proprietor: **MITSUI PETROCHEMICAL INDUS-TRIES, LTD.**
**2-5, Kasumigaseki 3-chome Chiyoda-ku Tokyo 100(JP)**

(72) Inventor: **Tanimoto, Shizufumi**
**2-6, Misono 1-chome**
**Otake-shiＳHiroshima 739-06(JP)**
Inventor: **Takahashi, Shigeru**
**2-7, Misono 1-chome**
**Otake-shi Hiroshima 739-06(JP)**

(74) Representative: **Myerscough, Philip Boyd et al J.A.Kemp & Co. 14, South Square Gray's Inn London, WC1R 5EU(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 276 575 B1

**Description**

The present invention concerns a method of multiplicating seedlings in a great amount by effecting tissue culture on plants in accordance with a specific method.

Vegetables such as cabbages, tomatoes, cucumbers and rices have been utilized for foods. Horticultural plants such as tulips, bluebattle and rudbeckia have been favoured as ornamental plants. These plants have been multiplicated so far by means of seeding or division of bulbs or tuber. However, such multiplication methods not only require a large area and much labour but also involve such problems as slow seedling growing rate or poor flower quality due to the spread of virus diseases. With the aim of overcoming these problems the technique of plant tissue culture has been used in recent years (for example see JP-A-55-14734). Multiplication by tissue culture has been attained by the differentiation of adventitious bud, adventitious embryo or bulb from cultured tissue pieces and cultured cells and its has been considered that the differentiation is controlled by the concentration ratio of cytokinin and auxin as plant hormone (for example, refer to Annals of Botany vol. 45, 321 - 327, 1980). However, there are many plant groups which do not undergo differentiation using plant hormones or the frequency of differentiation, if it occurs, is extremely low and there is a need for a more direct and effective method of inducing differentiation.

We have studied a method of more efficiently multiplicating seedlings of plants as compared with the usual case.

According to the invention there is provided a method of multiplicating seedlings of a plant wherein tissue culture is effected on tissue pieces or cultured cells of a plant belonging to Papaveraceae, Solanaceae except for Torenia, Umbelliferae, Rosaceae, Liliaceae except for Lilium, Compositae, Geraniaceae, Cucurbitaceae or Gramineae using a medium containing at least one calcium ionophore or cyclic AMP.

Specific examples of plants which can be used include those described in "Ground for the plants System Classification" edited by Yamagishi, published by Kokuryukan in 1974. More specifically, there can be mentioned plants belonging to Solanaceae such as egg plant, tomato, potato, sweet potato and basil, plants belonging to Papaveraceae such as poppy, rape, cabbage, radish and Chinese cabbage, plants belonging to Umbelliferae such as carrot, Japanese parsley and parsley, plants belonging to Rosaceae such as rose, strawberry, soybean and cherry, plants belonging to Liliaceae except for Lilium such as onions and tulips, plants belonging to Compositae such as chrysanthemum, bluebattle and sunflower, plants belonging to Geraniaceae such as pelargonium, geranium and flax, plants belonging to Cucurbitaceae such as cucumber and pumpkin, and plants belonging to Gramineae such as rice and corn. Among these plants relevant to the present invention, preferred plants are egg plant, potato, carrot, cabbage, onion, soybean, radish, basil, bluebattle rudbeckia, tulip, flax, cucumber and rice.

In the present invention, the tissue culture of plants can be conducted by using tissue pieces or cultured cells of the plant. The tissue pieces for culture can include, for example, tissue pieces of plants prepared by slicing cotyledon, hypocotyl, shoot apex, stalk, leaf, scale, root or other tissue. These tissue pieces are usually used after sterilization with sodium hypochlorite or ethanol. However, when using an aseptically cultured plant, a sterilized procedure is not required. Further, when multiplicating seedlings of plants with no disease or virus, tissues from near the apical point, that is tissue pieces of the plants obtained from tissue near the apical point can be used as the culture material. The cultured cells that can be used in the method of the present invention are not differentiated but are amorphous cells including callus tissue.

In the method of the present invention there is used a culture medium to which is added a calcium ionophore or cyclic-AMP (cyclic-adenosine-3'-5'-monophosphate). By this method, differentiation of the tissue or the cultured cells of a plant into an adventitious embryo or bulb is significantly promoted. The culture medium contains inorganic ingredients and a carbon source as essential ingredients, to which plant hormones and vitamins and, as required, amino acids can be added. The inorganic ingredients for the culture medium can include those inorganic salts including elements such as nitrogen, phosphor, potassium, sodium, calcium, magnesium, sulfur, iron, manganese, zinc, boron, molybdenum, chlorine, iodine and cobalts. Specifically, there can be mentioned those compounds such as potassium nitrate, sodium nitrate, ammonium nitrate, ammonium chloride, potassium chloride, calcium chloride, potassium monohydrogen phosphate, sodium dihydrogen phosphate, magnesium sulfate, magnesium chloride, sodium sulfate, ferrous sulfate, ferric sulfate, manganese sulfate, copper sulfate, sodium molybdate, molybdenum trioxide, potassium iodide, zinc sulfate, boric acid and cobalt chloride.

The carbon source for the culture medium can include, for example, carbon hydrate and derivatives thereof such as sucrose, organic acids such as fatty acid and primary alcohols such as ethanol.

2

Plant hormones for the culture medium can include, for example, auxins such as naphthalene acetic cid (NAA), indole acetic acid (IAA), p-chlorophenoxyacetic acid, 2,4-dichlorophenoxy acetic acid (2,4-D), indole acetic acid (IBA) and derivative thereof, and cytokinins such as benzyl adenine (BA), kinetin, zeatin, etc.

Vitamins for the culture medium can include, for example, biotine, thiamine (vitamin B1), pyridoxine (vitamin B6), pyridoxal, pyridoxamine, calcium pantotate, ascorbic acid (vitamin C), inositol, nicotinic acid, nicotinic amide and riboflavine (vitamin B2).

The amino acid for the culture medium can include, for example, glycine, alanine, glutamic acid, cystein, phenyl alanine and lysine.

The culture medium of the present invention is desirably used by usually incorporating from about 0.1 uM about 100 mM of the inorganic ingredient, from about 1 g/l to about 100 g/l of the carbon source, from about 0.01 mg/l to about 10 mg/l of the plant hormones, from about 0.1 mg/l to about 150 mg/l of the vitamin and from 0 to about 1000 mg/l of the amino acids.

The culture medium used for the tissue culture according to the present invention can include specifically those known culture media used for the tissue culture, for example, Murashige & Skoog culture medium ('62), Linsmaier & Skoog culture medium (RM-1965), White culture medium ('63), Gamborg B-5 culture medium, Mitsui M-9 culture medium, Nitch & Nitch culture medium, etc., being, preferably, incorporated as required with carbon sources and plant hormones as described above and, further, vitamins and amino acids as described above. The compositions of the known culture media mentioned above are described, for example, in "New Plant Tissue Culture" p386 - p391, written by Takeuchi, Nakajima, Furuya, and published from Asakura Shoten in 1979.

The culture medium used in the present invention is a liquid culture medium or solid culture medium usually containing from 0.5 to 1% of agar.

In the present invention, the concentration of calcium ionophore to be added in the culture medium suitably is from $10^{-8}$ to $10^{-4}$ M/l, preferably from $10^{-7}$ to $10^{-5}$ M/l. Among calcium ionophores, the use of A23187 is preferred. A23187 is 6S-(6 (2S*, 3S*), $8\beta(R^*)$, $9\beta$, 11)-5-(methylamino)-2-((3,9,11-trimethyl-8-(1-methyl-2-oxo-2-(1E-pyrrol-2-yl)ethyl)-1,7-dioxaspiro(5,5)-undec-2-yl)methyl)4-benzoxazolecarboxylic acid. Similarly, the concentration of the cyclic AMP suitably is from $10^{-9}$ to $10^{-5}$ M/l, preferably from $10^{-8}$ to $10^{-6}$ M/l.

In the present invention, the tissue pieces or cultured cells of plants as described above can be subjected to tissue culture using a liquid culture medium aerated with oxygen-containing gas in the same manner as described in JP-A-61-285928.

According to the method of the present invention, it is possible to obtain a large amount of adventitious buds, adventitious embryos or bulblets (small bulbs), at high efficiency from tissue pieces or cultured cells of plants. Adventitious buds and adventitious embryos obtained by the method according to the invention can be rooted to provide a plant body, which can then be sliced into tissue pieces (a bulblet can also be sliced) and subjected to further tissue culture in accordance with the present invention to enable seedlings to be prepared in a great amount. Further, plants obtained by the present invention can be grown into intact plant bodies of stable nature by usual cultivation.

Plant bodies of high quality can be obtained in a large amount by the method of the invention at a better efficiency as compared with conventional methods.

The present invention will now be described by reference to examples.

Examples 1 and 2

Tomato hypocotyl was sterilized in an agueous solution of 70% ethanol and sodium hypochlorite (effective chlorine content 1%) and then cut into about 1 cm length. Aseptic Murashige & Skoog agar medium (1962) (agar concentration 0.8%) at pH 5.6 containing 3% sucrose, $10^{-7}$ M naphthalene acetic acid, $10^{-6}$ M benzyl adenine and A23187 or cyclic AMP, at the concentration shown in Table 1 was prepared. The slices of tomato hypocotyl were added to the culture medium and cultured at 25°C in a bright place for 3 weeks. The results shown in Table 1 were obtained expressed as the number of adventitious bud formed per slice. In all of the treated slices, the number of adventitious bud obtained by differentiation was increased as compared with those in Comparative Example 1.

Comparative Example 1

Slices of tomato hypocotyl were subjected to tissue culture by the same procedure as in Example 1 except that the culture medium did not contain the A23187 or cyclic AMP. The results are also shown in Table 1.

## Table 1

| | Additive | Concentration | Number of adventitious bud/slice |
|---|---|---|---|
| Comparative Example 1 | not added | -- | 3.8 |
| Example 1 | A23187 | $10^{-6}M$ | 6.4 |
| " 2 | Cyclic AMP | $10^{-6}M$ | 5.0 |

Examples 3 and 4

Tissue culture was effected as in Example 1 except for using slices of egg plant hypocotyl with the results as shown in Table 2.

Examples 5 and 6

Tissue culture was effected as in Example 1 except for using slices of cabbage hypocotyl with the results shown in Table 2.

Examples 7 and 8

Tissue culture was effected as in Example 1 except for using slices of basil leaves with the results shown in Table 2.

Example 9

Tissue culture was effected as in Example 1 except for using slices of soybean leaves with the results shown in Table 2.

Example 10

Tissue culture was effected as in Example 1 except for using slices of radish hypocotyl with the results shown in Table 2.

Comparative Examples 2 to 6

Tissue culture was effected on slices of egg plant hypocotyl, slices of cabbage hypocotyl, basil leaves, slice of soybean leaves and slices of radish hypocotyl as in Examples 3 to 10 except that the culture medium did not contain A23187 or cyclic AMP. The results are also shown in Table 2.

4

Table 2

| | Material | Additive | Concentration | Number of adventitious bud/slice |
|---|---|---|---|---|
| Comparative Example 2 | Slice of egg plant hypocotyl | not-added | — | 3.2 |
| " 3 | slice of cabbage hypocotyl | " | — | 1.2 |
| " 4 | slice of basil leaves | " | — | 3.2 |
| " 5 | slice of soybean leaves | " | — | 0 |
| " 6 | slice of radish hypocotyl | " | — | 0 |
| Example 3 | slice of egg plant hypocotyl | A23187 | $10^{-6}$ M | 5.8 |
| " 4 | " | Cyclic AMP | $10^{-6}$ M | 4.0 |
| " 5 | slice of cabbage hypocotyl | A23187 | $10^{-6}$ M | 3.4 |
| " 6 | " | Cyclic AMP | $10^{-6}$ M | 2.6 |
| " 7 | slice of basil leaves | A23187 | $10^{-6}$ M | 7.4 |
| " 8 | " | Cyclic AMP | $10^{-6}$ M | 8.4 |
| " 9 | slice of soybean leaves | A23187 | $10^{-5}$ M | 1.2 |
| " 10 | slice of radish hypocotyl | A23187 | $10^{-6}$ M | 1.0 |

Examples 11 and 12

Tissue culture was effected as in Example 1 except for using slices of bluebattle with the results shown in Table 3.

5

Examples 13 and 14

The same procedures were conducted as in Example 1 except for using slices of rudbeckia stalk with the results shown in Table 3.

Example 15

The same procedures were conducted as in Example 1 except for using callus cells of tomato with the results shown in Table 3.

Example 16

The same procedures were conducted as in Example 1 except for using callus cells of egg plant with the results shown in Table 3.

Comparative Examples 7 to 10

Tissue culture was effected on slices of bluebattle leaves, slices of rudbeckia leaves, callus cells of tomatoes and callus cells of egg plant as in Examples 11 to 16 except that the culture medium did not contain A23187 or cyclic AMP. The results are also shown in Table 3.

Table 3

| | Material | Additive | Concentration | Number of adventitious bud/slice |
|---|---|---|---|---|
| Comparative Example 7 | slice of bluebottle leaves | not-added | — | 2.2 |
| " 8 | slice of rudbeckia leaves | " | — | 2.5 |
| " 9 | tomato callus cell | " | — | 3.4 |
| " 10 | Egg plant callus cell | " | — | 3.0 |
| Example 11 | slice of bluebottle leaves | A23187 | $10^{-5}$M | 7.6 |
| " 12 | " | Cyclic AMP | $10^{-6}$M | 6.6 |
| " 13 | slice of rudbeckia leaves | A23187 | $10^{-5}$M | 5.6 |
| " 14 | " | Cyclic AMP | $10^{-6}$M | 4.2 |
| " 15 | tomato callus cell | A23187 | $10^{-6}$M | 7.8 |
| " 16 | egg plant callus cell | A23187 | $10^{-6}$M | 5.6 |

Examples 17 and 18

The same procedures were conducted as in Example 1 except for using slices of scaly leaves of onion attached to a disk of onions with the results shown in Table 4.

Examples 19 and 20

The same procedures were conducted as in Example 1 except for using slices of potato leaves with the results shown in Table 4.

Examples 21 and 22

The same procedures were conducted as in Example 1 except for using slices of flax hypocotyl with the results shown in Table 4.

Example 23

The same procedures were conducted as in Example 1 except for using tulip slices of scales with the results shown in Table 4.

Examples 24 and 25

The same procedures were conducted as in Example 1 except for using slices of asparagus stalks with the results shown in Table 4.

Comparative Examples 11 to 15

Tissue culture was effected on slices of onion scaly leaves, slices of potato leaves, slices of flax hypocotyl slices of tulip scaly leaves and slices of asparagus stalk in the same procedures as in Examples 17 to 25 except that the culture medium did not contain A23187 or cyclic AMP. The results are also shown in Table 4.

Table 4

| | Material | Additive | Concentration | Number of adventitious bud/slice |
|---|---|---|---|---|
| Comparative Example 11 | slice of onion scaly leaves | not added | — | 6.8 |
| " 12 | slice of potato leaves | " | — | 2.2 |
| " 13 | slice of flax hypocotyl | " | — | 6.4 |
| " 14 | slice of tulip scale | " | — | 4.2 |
| " 15 | slice of asparagus stalk | " | — | 0.6 |
| Example 17 | slice of onion scaly leaves | A23187 | $10^{-6}$M | 12.8 |
| " 18 | " | Cyclic AMP | $10^{-6}$M | 9.6 |
| " 19 | slice of potato leaves | A23187 | $10^{-6}$M | 6.8 |
| " 20 | " | cyclic AMP | $10^{-6}$M | 5.4 |
| " 21 | slice of flax hypocotyl | A23187 | $10^{-6}$M | 14.6 |
| " 22 | " | Cyclic AMP | $10^{-6}$M | 12.4 |
| " 23 | slice of tulip scale | A 23187 | $10^{-6}$M | 8.8 |
| " 24 | slice of asparagus stalk | A23187 | $10^{-6}$M | 3.6 |
| " 25 | " | Cyclic AMP | $10^{-6}$M | 3.4 |

Examples 26 and 27

Carrot hypocotyl was sterilized in an aqueous solution of 70% ethanol and sodium hypochlorite (1% effective chlorine amount ) and cut into about 1 cm length. Aseptic Murashige - Skoog liquid medium (1962) at pH 5.6 containing 4% sucrose, $10^{-6}$ M 2,4-chlorophenoxy acetic acid and A23187 or cyclic AMP at a concentration shown in Table 5 was prepared. The slices of carrot hypocotyl were added and cultured at 25°C in a dark place for three weeks with the results shown in Table 5.

Comparative Example 16

Slices of carrot hypocotyl were cultured in a culture medium containing no plant hormone for two weeks and the number of adventitious embryoes formed per slice was as shown in Table 5.

Table 5

| | Material | Additive | Concentration | Number of Adventitious bub/slice of explant (0.1g) |
|---|---|---|---|---|
| Comparative Example 16 | slice of carrot hypocotyl | not added | – | 2.4 |
| Example 26 | slice of carrot hypocotyl | A23187 | $10^{-6}$M | 4.2 |
| Example 27 | " | Cyclic AMP | $10^{-6}$M | 3.8 |

## Claims

1.  A method of multiplicating seedlings of a plant wherein tissue culture is effected on tissue pieces or cultured cells of a plant belonging to Papaveraceae, Solanaceae except for Torenia, Umbelliferae, Rosaceae, Liliaceae except for Lilium, Compositae, Geraniaceae, Cucurbitaceae or Gramineae using a medium containing at least one calcium ionophore or cyclic AMP.

2. A method as defined in claim 1, wherein the calcium ionophore is A23187.

## Revendications

1. Procédé de multiplication des plants d'une plante, dans lequel on effectue une culture de tissus sur des morceaux de tissus ou sur des cellules cultivées d'une plante faisant partie des papavéracées, des solanacées excepté Torenia, des ombellifères, des rosacées, des liliacées excepté Lilium, des composées, des géraniacées, des cucurbitacées ou des graminées, en utilisant un milieu contenant au moins un ionophore du calcium ou de l'AMP-cyclique.

2. Procédé tel que défini dans la revendication 1, dans lequel l'ionophore du calcium est le composé A23187.

## Patentansprüche

1. Verfahren zum Vermehren von Pflanzensetzlingen, bei dem Gewebe auf Gewebsstücken oder kultivierten Zellen einer Pflanze, die zu Papaveraceae, Solanaceae, ausgenommen Torenia, Umbelliferae, Rosaceae, Liliaceae, ausgenommen Lilium, Compositae, Geraniaceae, Cucurbitaceae oder Gramineae gehört, kultiviert wird, unter Verwendung eines Mediums, das mindestens ein Calciumionophor oder cyclisches AMP enthält.

2. Verfahren nach Anspruch 1, worin das Calciumionophor A23187 ist.